# EUROPÄISCHE PATENTANMELDUNG

(11) **EP 1 935 391 A1**
(43) Veröffentlichungstag der Anmeldung: **25.06.2008**
(21) Anmeldenummer: 07023626.0
(22) Anmeldetag: 06.12.2007
(51) Int. Cl.: A61H 15/00, A61H 23/02, A61N 5/06, A61H 15/02

(54) **Vorrichtung zur therapeutischen und/oder kosmetischen Behandlung einer Körperzone**

(30) Priorität: 22.12.2006 DE 202006019324 U
(71) Anmelder: Biro, Stephan, 44141 Dormund (DE)
(72) Erfinder: Biro, Stephan, 44141 Dormund (DE)
(74) Vertreter: Vomberg, Friedhelm

(57) **Zusammenfassung**

Die Erfindung betrifft eine Vorrichtung zur therapeutischen und/oder kosmetischen Behandlung einer Körperzone mit einer Bestrahlungseinheit zur Licht- und/oder Wärmebehandlung und einer Massageeinheit. Um Synergieeffekte nutzbar zu machen und den Erholungs- und Entspannungseffekt zu optimieren wird vorgeschlagen, die Bestrahlungseinheit (1) und die Massageeinheit (2) zeitgleich auf die Körperzone einwirken zu lassen.

## Beschreibung

Gegenstand der Erfindung ist eine Vorrichtung zur therapeutischen und/oder kosmetischen Behandlung einer Körperzone mit einer Bestrahlungseinheit und einer Massageeinheit, wobei die Bestrahlungseinheit zur Licht- und/oder Wärmebehandlung einsetzbar ist.

Zur Licht- und/oder Wärmebehandlung werden überwiegend die kurzwellige Ultraviolettstrahlung (UV-Strahlung) und die langwellige Infrarotstrahlung (IR-Strahlung) eingesetzt. Die Bräunungswirkung der UV-Strahlung ist allgemein bekannt und findet beispielsweise bei Solarien Anwendung. Die IR-Strahlung, welche auch als Wärmestrahlung bezeichnet wird, wird überwiegend im medizinischen Bereich eingesetzt, beispielsweise zur örtlichen Behandlung von Entzündungen. Des Weiteren gilt es als erwiesen, dass Licht, insbesondere farbiges Licht, eine positive Wirkung auf die menschliche Psyche haben kann. Aus dem Wellnessbereich ist beispielsweise der Begriff der Farblichttherapie bekannt. Insofern ist auch die Strahlung einer anderen Wellenlänge als der von UV- oder IR-Strahlung zur therapeutischen und/oder kosmetischen Behandlung einsetzbar.

Vorrichtungen zur Bestrahlung findet man - je nach Zweck der Behandlung - u.a. in Arztpraxen, Fitness-, Kosmetik- oder Sonnenstudios. So umfasst heute die Ausstattung eines Sport- oder Fitnessstudios regelmäßig auch ein Solarium, dessen UV-Strahlung einerseits eine Bräunung der Haut bewirken soll, während Licht und Wärme zur Entspannung des Körpers nach einer anstrengenden sportlichen Betätigung beitragen sollen.

Zur Ganzkörperbestrahlung sind Solarien in der Regel mit einer Liegefläche und einer darüber angeordneten höhenverstellbaren und/oder verschwenkbaren Haube ausgestattet, wobei UV-Röhren oder Strahler in der Liegefläche sowie in der Haube integriert sind und somit eine allseitige gleichmäßige Bräunung des Körpers ermöglichen. Die Bestrahlungsdauer beträgt je nach Geräteleistung in der Regel 15 bis 30 Minuten.

Etwa dieselbe Zeitdauer beansprucht eine Teilkörpermassage, bei der über die lokale mechanische Beeinflussung von Haut, Bindegewebe und Muskulatur durch Dehnungs-, Zug- und Druckreize eine Entspannung der Muskulatur und eine Verbesserung des Zellstoffwechsels im Gewebe erreicht werden können. Um die Wirkungen zu verstärken, geht der Massage oftmals eine Wärmebehandlung voraus. Hierzu wird beispielsweise erwärmter Mineralschlamm (Fango) direkt oder indirekt auf die später zu massierende Stelle des Körpers aufgetragen. Die Dauer einer solchen Behandlung liegt zwischen 20 und 40 Minuten, wobei die Wärme tief in das Körpergewebe eindringen kann und dieses somit lang anhaltend und wirkungsvoll erwärmt.

Mit der körperlichen Entspannung geht regelmäßig auch eine psychische Entspannung einher, so dass der Erfolg einer Behandlung am allgemeinen Wohlbefinden der behandelten Person ablesbar ist. Insofern dienen Bestrahlung sowie jede andere Art der Wärmebehandlung und Massage letztendlich demselben Zweck, nämlich das allgemeine Wohlbefinden zu steigern.

Aufgrund der gleichen Zielsetzung und im Hinblick auf eine damit verbundene mögliche Verkürzung der Behandlungsdauer, erscheint es daher sinnvoll, die verschiedenen Behandlungsmethoden zu kombinieren, d.h. zeitgleich auszuführen. Beispielsweise könnte eine Massagebehandlung unter Wärmeeinwirkung Synergieeffekte nutzbar machen, die zu verbesserten Behandlungserfolgen führen würden.

Eine Vorrichtung, welche die zeitgleiche Durchführung einer Bestrahlung und einer Massage ermöglichen soll, wird in der DE 32 46 436 A1 beschrieben. Vorgeschlagen wird eine Liegefläche mit einer Solarium-Bestrahlung, wobei über einer schwimmenden Liegematte als Liegefläche ein höhenverstellbarer Solarhimmel angebracht ist. Die Vorrichtung soll insbesondere eine Verbesserung des Erholungseffektes der Solarium-Bestrahlung bewirken, indem der Liegekomfort verbessert wird. Darüber hinaus wird vorgeschlagen, während der Bestrahlung über elektromotorisch erzeugte Wasserstöße den auf der Liegefläche aufliegenden Körper zu massieren. Der Nachteil dieser Vorrichtung besteht u.a. darin, dass der mittels Wasserstöße erzeugte Druckreiz für eine effektive Massage zu gering ist, insbesondere wenn zwischen Wasseroberfläche und Körper eine Liegematte angeordnet ist.

Aufgabe der vorliegenden Erfindung ist es eine verbesserte Vorrichtung mit einer Bestrahlungs- und einer Massageeinheit zu schaffen, um Synergieeffekte nutzbar zu machen und den Erholungs- und Entspannungseffekt dadurch weiter zu optimieren.

Zur Lösung dieser Aufgabe wird eine Vorrichtung mit den Merkmalen nach Anspruch 1 vorgeschlagen. Konkrete Ausführungsformen werden in den Unteransprüchen beschrieben.

Erfindungsgemäß wirken die Bestrahlungseinheit und die Massageeinheit der zur therapeutischen und/oder kosmetischen Behandlung einer Körperzone vorgesehenen Vorrichtung zeitgleich auf diese Körperzone ein, d.h., dass eine Körperzone oder ein Bereich eines Körpers gleichzeitig massiert und bestrahlt werden kann. Besteht ein Anteil der Strahlung beispielsweise aus IR-Strahlung, kann über die Wärmewirkung der Strahlung die Effektivität der Massage erhöht werden. Der Einsatz von UV-Strahlern in der Bestrahlungseinheit bewirkt zudem eine Bräunung der Haut an der massierten Stelle oder, sofern die Bestrahlungseinheit entsprechend ausgelegt ist, am ganzen Körper.

Bevorzugt umfasst die Vorrichtung eine Liege- oder Sitzfläche, in der die Bestrahlungseinheit und die Massageeinheit integriert sind. Die Behandlung kann somit in einer Ruheposition des zu behandelnden Körpers erfolgen, wodurch der Entspannungseffekt weiter gesteigert wird. Die Liege- oder Sitzfläche weist hierzu mindestens einen elastischen und/oder strahlungsdurchlässigen Bereich auf, hinter dem die Bestrahlungseinheit und die Massageeinheit ganz oder zumindest teilweise angeordnet sind. Insbesondere kann die Bestrahlungseinheit derart angeordnet sein, dass der ganze Körper einer liegenden oder sitzenden Person von der Bestrahlung erfasst wird. Der Wirkungsbereich der Massageeinheit wird dagegen von dem elastischen Bereich der Liege- oder Sitzfläche festgelegt. Vorzugsweise befindet sich zumindest ein elastischer Bereich im Rücken und/oder Schulterbereich, soweit die Körperlage durch die Liege- oder Sitzfläche vorgegeben ist. Der Liege- oder Sitzbereich kann beispielsweise ergonomisch geformt sein und somit eine bestimmte Liege- oder Sitzposition vorgeben. Bei einer Liege ist insbesondere eine Anhebung der Liegefläche im Fuß- und Unterschenkelbereich über ein Fußteil empfehlenswert, da der Rücken einer liegenden Person hierbei gestreckt und die Wirbelsäule entlastet wird. Zudem wird einer Hohlkreuzbildung entgegengewirkt. Der oder die elastischen Bereiche sollten vorzugsweise auch strahlungsdurchlässig sein, damit erfindungsgemäß neben der Massageeinheit auch die Bestrahlungseinheit auf die jeweilige Körperzone einwirken kann.

Die Bestrahlungseinheit umfasst mindestens eine strahlungsemittierende Röhre und/oder mindestens einen Strahler, die bzw. der beweglich gelagert und/oder auf die zu behandelnde Körperzone ausrichtbar ist. Vorzugsweise sind die Röhren und/oder Strahler zumindest in eine Richtung verschiebbar und/oder drehbar bzw. schwenkbar angeordnet. Ferner bevorzugt beinhaltet die Bestrahlungseinheit herkömmliche UV-Röhren und/oder Strahler, wie sie in Solarien vorzufinden sind, um während der Massagebehandlung eine Bräunung der Haut an der massierten Stelle bewirken zu können. Um eine Ganzkörperbräunung zu ermöglichen, sollten die Röhren und/oder Strahler derart angeordnet sein, dass die gesamte Liege- oder Sitzfläche von der Strahlung erfasst wird. Zudem ist oberhalb der Liege- oder Sitzfläche eine Haube oder ein Schirm mit zusätzlichen UV-Röhren und/oder Strahlern anzuordnen, wobei als Haube oder Schirm das Oberteil einer gewöhnlichen Sonnenbank dienen kann.

Die Massageeinheit umfasst mindestens ein beweglich gelagertes und/oder auf die zu behandelnde Körperzone ausrichtbares Massageglied, mittels dessen Druck auf die zu behandelnde Körperzone ausübbar ist. Vorzugsweise wird der Druck über mehrere ballen- oder kugelförmige Körper auf die zu massierende Körperzone übertragen, indem die ballen- oder kugelförmigen Körper einzeln oder gruppenweise in linearen und/oder kreisenden Bewegungen über die Körperzone geführt werden. Da der menschliche Körper nicht einheitlich geformt ist und jeder Mensch zudem ein anderes Schmerzempfinden besitzt, bestehen die ballen- oder kugelförmigen Körper der Massageeinheit vorzugsweise aus einem elastischen Material, beispielsweise Gummi oder Silikon, und sind zudem federnd gelagert, um Unebenheiten der Körperzone auszugleichen sowie einen gleichmäßigen Anpressdruck zu gewährleisten. Der Anpressdruck kann ferner durch Anheben oder Absenken der Massageglieder verändert werden und ist somit auf die zu behandelnde Person einstellbar. Weiterhin bevorzugt wird der Massagedruck mittels Drucksensoren überwacht, und gegebenenfalls automatisch korrigiert, sofern ein voreingestellter, individueller maximaler Massagedruck überschritten wird. Ferner kann durch seitliches Verschieben oder Verschwenken (Veränderung des Anstellwinkels) das Massageglied auf eine bestimmte Stelle der Körperzone ausgerichtet werden.

Jedes Massageglied ist mit mindestens einer elektrischen Antriebseinheit verbunden, mittels derer das Massageglied in lineare, insbesondere rollende oder klopfende und/oder kreisende und/oder oszillierende Bewegungen versetzbar ist. Dabei kann vorgesehen sein, dass die Massageglieder einzeln oder gruppenweise nur bestimmte Bewegungen ausführen können und gegebenenfalls abwechselnd oder nacheinander eingesetzt werden. Bevorzugt ist jedoch jedes Massageglied in jeder der vorstehend genannten Arten bewegbar. Weiterhin bevorzugt ist die elektrische Antriebseinheit mit einer programmierbaren Steuerung verbunden, mittels derer individuelle Programmabläufe hinsichtlich der Bewegung der einzelnen Massageglieder voreingestellt und später ausgewählt werden können. Durch vibrierende Massageglieder kann die Massagewirkung zudem erhöht werden.

Um zeitgleich auf die zu behandelnde Körperzone einwirken zu können und eine gleichmäßige Bestrahlung gewährleisten zu können, sind die Röhren und/oder Strahler der Bestrahlungseinheit bevorzugt neben und/oder unter und/oder zwischen den Massagegliedern angeordnet. Zur Ausrichtung, d.h. zum Verschieben und/oder Verschwenken der Massageeinheit müssen daher gegebenenfalls auch die Elemente der Bestrahlungseinheit verschoben werden. Hierzu sind die Röhren und/oder Strahler auf einem oder mehreren verschiebbar gelagerten Trägern angeordnet und können bevorzugt gruppenweise auseinander geschoben werden. Mitnehmer an den beweglich gelagerten Elementen der Massageeinheit, die in Eingriff mit einer Ausnehmung an dem oder den Trägern stehen, gewährleisten, dass das Verschieben der Röhren und/oder Strahler gleichzeitig mit der Bewegung der Massageglieder erfolgt. Zur Führung des Trägers oder der Träger können Gleitschienen vorgesehen sein.

Die bevorzugt ballen- oder kugelförmigen Massageglieder sind abnehmbar. Dies erleichtert den Austausch der unterhalb der Massageglieder angeordneten Röhren und/oder Strahler. Zudem sind Massageglieder in unterschiedlichen Ausführungsarten vorgesehen, so dass Formen, Oberflächen (glatt oder strukturiert, beispielsweise mit Noppen) und Festigkeiten individuell auswählbar sind.

Je nach Lage der auf die zu behandelnde Körperzone ausgerichteten Massageglieder, Röhren und/oder Strahler können konstruktionsbedingt Bereiche geringerer Strahlungsintensität entstehen, beispielsweise durch Verschattung oder sich wegen des Verschiebens der Röhren und/oder Strahler bildender Lücken. Um diese Lücken auszugleichen, ist vorzugsweise im strahlungsdurchlässigen Bereich der Liege- oder Sitzfläche zusätzlich mindestens eine breit abstrahlende Strahlerleiste angeordnet, deren Strahlung auch die Bereiche geringerer Strahlungsintensität erreicht. Bevorzugt ist die zusätzliche breit abstrahlende Strahlerleiste mittig in Bezug auf die Liege- oder Sitzfläche der Vorrichtung angeordnet und muss nicht zwecks Ausrichtung der Massageglieder verschoben werden.

Aus hygienischen Gründen ist die Oberfläche der Liege- oder Sitzfläche der Vorrichtung derart auszuführen, dass sie leicht zu reinigen und zu desinfizieren ist. Aufgrund der Wärmestrahlung ist zudem mit einer erhöhten Transpiration der behandelten Person zu rechnen, so dass die Liege- oder Sitzfläche vorzugsweise feuchtigkeitsunempfindlich auszuführen ist. Die Liege- oder Sitzfläche sollte zudem den Körper stützen. Bevorzugt besteht daher die Liege- oder Sitzfläche aus einer ergonomisch geformten, strahlungsdurchlässigen festen Kunststoffschale, die im Wirkungsbereich der Massageeinheit, nämlich dem elastischen und/oder strahlungsdurchlässigen Bereich, ausgespart ist. Wobei in der Liege- oder Sitzfläche auch mehrere solcher Bereiche vorgesehen sein können.

Vorzugsweise besteht mindestens ein elastischer und/oder strahlungsdurchlässiger Bereich der Liege- oder Sitzfläche aus einer abnehmbaren Folie, die feuchtigkeitsundurchlässig mit der Liege- oder Sitzfläche verbunden ist. Diese Folie sollte besonders reißfest und - wie die Kunststoffschale - leicht zu reinigen und zu desinfizieren sein. Darüber hinaus ist sie derart mit der Liege- oder Sitzfläche zu verbinden, dass sie leicht abnehmbar und somit schnell zu wechseln ist. Bevorzugt wird daher eine Klemmverbindung vorgeschlagen, wobei es die Bildung eines Wulstes oder eines Steges auf der Liege- oder Sitzfläche zu vermeiden gilt, um einerseits den Liegekomfort nicht zu beeinträchtigen, andererseits die Reinigung nicht zu erschweren.

Vorzugsweise ist um den elastischen und/oder strahlungsdurchlässigen Bereich in der Liege- oder Sitzfläche eine Ausnehmung zur Aufnahme des Folienrandes angeordnet, wobei zur Fixierung des Folienrandes zusätzlich eine mit Druckluft beaufschlagbare Schlauchringleitung in die Ausnehmung einlegbar ist. Die Druckluft presst die Schlauchringleitung gegen die Innenwand der Ausnehmung, die vorzugsweise einen im Wesentlichen kreisförmigen Querschnitt besitzt, so dass der in die Ausnehmung eingelegte Folienrand zwischen Schlauchringleitung und Innenwand der Ausnehmung eingeklemmt wird. Der Querschnitt der Schlauchringleitung ist im entspannten Zustand kleiner als der Querschnitt der Ausnehmung, so dass dieser ohne Beaufschlagung mit Druckluft zur Abnahme der Folie leicht entnommen werden kann. Über die Schlauchringleitung ist zudem eine dichte Verbindung gewährleistet.

Die Vorteile der vorstehend beschriebenen Vorrichtung bestehen somit insbesondere in einer Steigerung der Effektivität der Behandlung, während die Behandlungsdauer gegenüber zeitlich nacheinander vollzogener Behandlungsschritte erheblich reduziert werden kann.

Eine Steigerung der Entspannungswirkung kann zusätzlich durch akustische, visuelle und/oder olfaktorische Reize erzielt werden. Beispielsweise kann eine Beschallung mit ruhiger Musik über in die Vorrichtung integrierte Lautsprecher oder Kopfhörer vorgesehen sein. Zudem könnte mittels einer Brille, die visuelle Signale, wie Farben, Muster etc., abgibt, beruhigend auf die zu behandelnde Person eingewirkt werden. Ferner könnte die Raumluft mit besonderen Aroma- und/oder Duftstoffen versetzt werden.

Ein Ausführungsbeispiel der erfindungsgemäßen Vorrichtung ist in den Zeichnungen dargestellt:
- Fig. 1: eine Draufsicht auf die Liegefläche einer erfindungsgemäßen Vorrichtung (bei Einstellung des maximalen Massagebereichs);
- Fig. 2: einen Längsschnitt durch die Liegefläche nach Fig. 1;
- Fig. 3: eine Draufsicht auf die Liegefläche (bei Einstellung des minimalen Massagebereichs);
- Fig. 4: einen Querschnitt durch die Liegefläche nach Fig. 1;
- Fig. 5: einen Querschnitt durch die Liegefläche nach Fig. 3;
- Fig. 6: eine Vergrößerung des Querschnitts nach Fig. 5;
- Fig. 7: einen Querschnitt durch die Massageeinheit;
- Fig. 8: eine Draufsicht auf die Massageeinheit;
- Fig. 9: eine Draufsicht auf die Massageeinheit bei veränderter Position der Massageglieder;
- Fig. 10: einen Längsschnitt durch die Massageeinheit gemäß Fig. 9;
- Fig: 11: eine Draufsicht auf den elastischen, strahlungsdurchlässigen Bereich der Liegefläche;
- Fig. 12: einen Detailschnitt durch die Schlauchringbefestigung mit entspanntem Schlauch;
- Fig. 13: einen Detailschnitt durch die Schlauchringbefestigung mit druckbeaufschlagtem Schlauch;
- Fig. 14: eine schematische Darstellung des Bewegungsablaufes der Massageglieder:
a) Grundstellung,
b) 45° Stellung;
- Fig. 15: eine schematische Darstellung des Bewegungsablaufes der Massageglieder:
a) 45° Stellung,
b) Grundstellung;
Fig. 16 eine schematische Darstellung des Bewegungsablaufes der Massageglieder: Ruhestellung

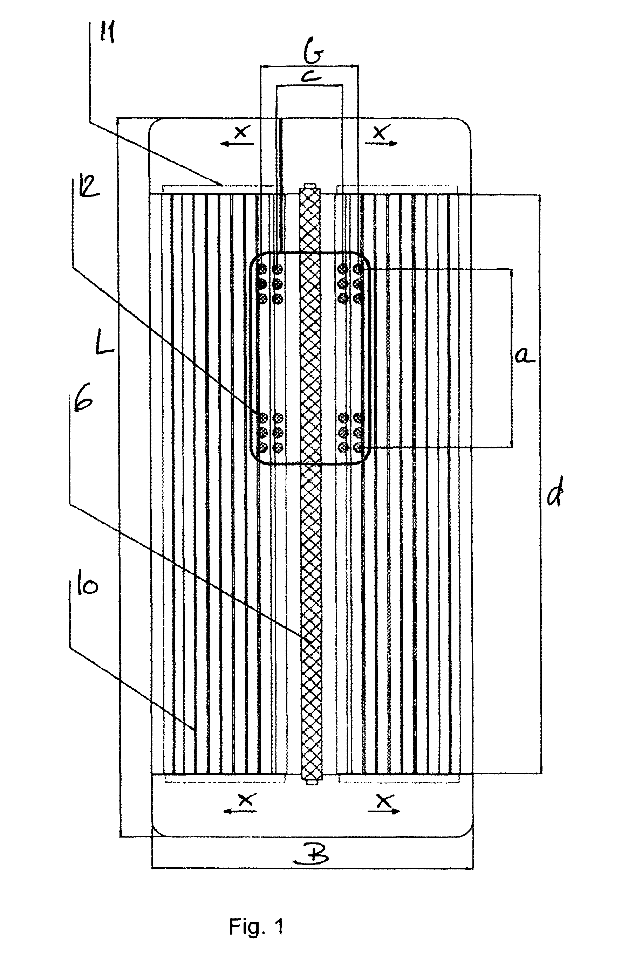

Fig. 1 bis 16 zeigen die erfindungsgemäße Vorrichtung in der Ausführung als Solarium mit einer in der Liegefläche 3 des Solariums integrierten Massageeinheit 2. Das Solarium besitzt die für eine Sonnenbank typische Ausgestaltung, d.h. es weist eine mit UV-Röhren 10 ausgestattete Liegefläche 3 und eine darüber angeordnete, ebenfalls mit UV-Röhren 10 ausgestattete höhenverstellbare und/oder verschwenkbare Haube auf. Im Unterschied zu herkömmlichen Sonnenbänken ist in die Liegefläche 3 zusätzlich eine Massageeinheit 2 integriert. Da die Haube keine besonderen Merkmale aufweist wird auf die Darstellung und eine detaillierte Beschreibung verzichtet.

Der Draufsicht in Fig. 1 ist zu entnehmen, dass unterhalb eines strahlungsdurchlässigen Bereichs der Liegefläche 3 mehrere, eine Bestrahlungseinheit 1 bildende UV-Röhren 10 parallel zur Längsachse der Liegefläche 3 angeordnet sind, wobei mehrere rechts von der Längsachse angeordnete UV-Röhren 10 eine erste Einheit bilden und mehrere links von der Längsachse eine weitere Einheit bilden. Beide Bestrahlungseinheiten sind quer zur Längsachse verschiebbar angeordnet (siehe Pfeile x). Hierzu weist jede Bestrahlungseinheit mindestens zwei Röhrenträger 14 auf, die auf quer zur Röhrenlängsachse angeordneten Führungsschienen 11 gelagert sind. Im Bereich der Längsachse ist eine UV- Strahlerleiste 6 als weitere Bestrahlungseinheit angeordnet, die im Unterschied zu den UV-Röhren nicht beweglich gelagert ist.

Die Liegefläche 3, die entsprechend Fig. 1 die Außenabmessungen Länge L = 2200 mm und Breite B = 930 mm besitzt, weist einen in Bezug auf die Breite B mittig und in Bezug auf die Länge L nach oben versetzten, elastischen und strahlungsdurchlässigen Bereich 4 auf. Unterhalb dieses Bereiches ist eine Massageeinheit 2 mit 24 kugelförmigen Massagegliedern 12 angeordnet, wobei die Massageglieder 4 Gruppen von jeweils 6 Massagekugeln bilden, deren Lage zueinander veränderbar ist. Über die Lage der Massagekugeln wird der Massagebereich definiert. Die in Fig. 1 dargestellte Stellung der Massagekugeln zeigt den maximalen Massagebereich, bei dem alle 4 Gruppen den größten Abstand zueinander aufweisen. In Längsrichtung beträgt der Achsabstand a der außen liegenden Massagekugeln 545 mm, in Querrichtung beträgt der Achsabstand b der außen liegenden Massagekugeln 280 mm und der Achsabstand c der innen liegenden Massagekugeln 190 mm.

Damit die Massageglieder 12 die in Fig. 1 dargestellte Position einnehmen können, müssen die UV-Röhren 10 nach außen (in Pfeilrichtung x) verschoben werden. Hierdurch entsteht entlang der Längsachse der Liegefläche 3 ein Bereich, in dem keine UV-Röhren 10 angeordnet sind. Dieser Bereich wird von der mittig angeordneten Strahlerleiste 6 erfasst, deren Strahler derart angeordnet sind, dass sie in einem Winkel abstrahlen, der keine Bestrahlungslücke entstehen lässt. Im gesamten strahlungsdurchlässigen Bereich der Liegefläche 3 ist somit eine Bestrahlung gleicher Strahlungsintensität gewährleistet. Die Breite des strahlungsdurchlässigen Bereichs entspricht der Breite B der Liegefläche, während die Länge d des strahlungsdurchlässigen Bereichs mit 1770 mm kürzer als die Liegefläche ist.

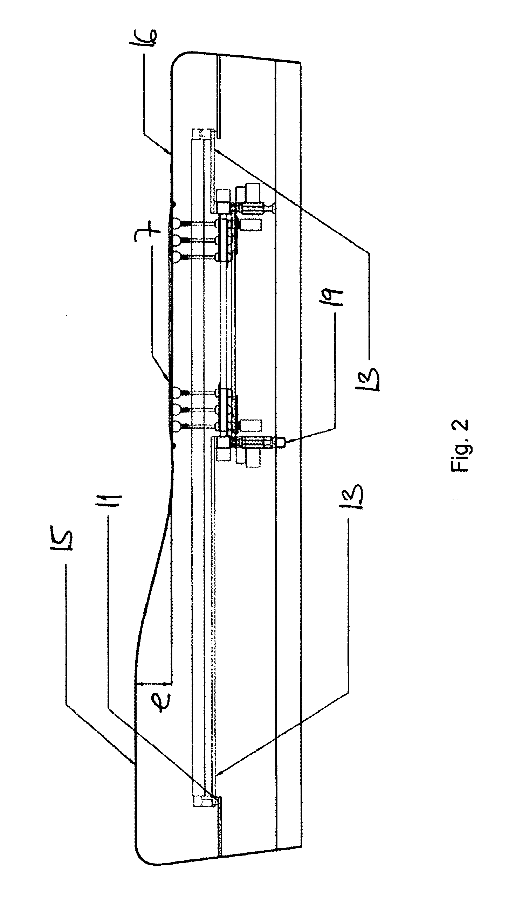

Fig. 2 zeigt einen Längsschnitt durch die Liegefläche 3 entsprechend Fig. 1, d.h. mit den Massagegliedern 12 in der maximal auseinander gefahrenen Stellung. Dem Umriss ist zu entnehmen, dass die Liegefläche 3 ergonomisch geformt ist, indem der Fußbereich um das Maß e = 80 mm angehoben ist. Das höher liegende Fußteil 15 sorgt für eine entspannte Körperlage, bei der der Rücken gestreckt wird und auf der Massagefläche zu liegen kommt. Das Kopfteil 16 ist waagerecht ausgeführt, wobei wahlweise ein strahlungsdurchlässiges Luftkissen als Auflage verwendet werden kann.

Zwischen Fußteil 15 und Kopfteil 16 ist der elastische und strahlungsdurchlässige Bereich 4 angeordnet. Hierzu weist die aus einem strahlungsdurchlässigen bzw. transparenten Kunststoff bestehende formstabile Liegefläche 3 eine Aussparung auf, welche mit einer ebenfalls strahlungsdurchlässigen, elastischen und zudem reißfesten Folie 7 abgedeckt ist. Die Folie 7 ist geringfügig größer als die Aussparung in der Liegefläche 3. Die überstehenden Folienränder werden umlaufend in einer Ausnehmung 8 der Liegefläche 3 über eine mit Druckluft beaufschlagte Schlauchringleitung 9 gehalten. Die Verbindung der Folie 7 mit der Liegefläche 3 ist somit feuchtigkeitsundurchlässig ausgeführt. Die Abdeckung aus Folie gewährleistet, dass die Massageglieder 12 auf die zu massierende Körperzone den erforderlichen Massagedruck ausüben können, während die formstabile Kunststoffschale der Liegefläche 3 den Körper stützt.

Dem Längsschnitt der Fig. 2 sind die weiteren Bestandteile der Massageeinheit 2 zu entnehmen. Direkt unterhalb der Folie 7 sind die 24 Massagekugeln der Massageeinheit 2 angeordnet, von denen jede dreh-, schwenk- sowie vertikal und horizontal verschiebbar gelagert ist. Jeweils 6 Massagekugeln bilden eine Gruppe. Gemäß dem dargestellten Ausführungsbeispiel werden die Massagekugeln gruppenweise bewegt und auf die zu massierende Körperzone ausgerichtet. Jede Gruppe weist hierzu eine entsprechende Halterung und Führung auf, an denen Mitnehmer 13 angeordnet sind, die in eine Ausnehmung der Röhrenträger 14 greifen und diese mitbewegen. Dadurch ist sichergestellt, dass die UV-Röhren 10 die Bewegungen der Massagekugeln nicht behindern.

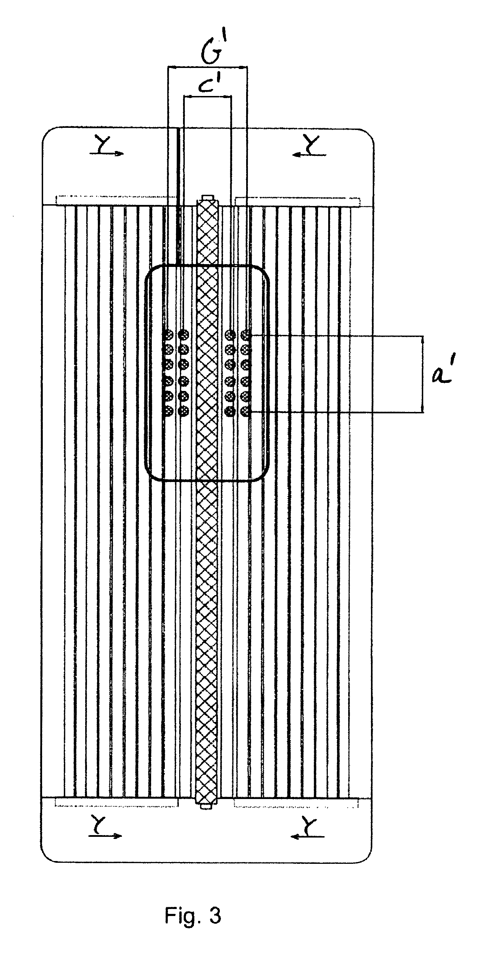

Eine andere Stellung der Massagekugel zeigt die Draufsicht in Fig. 3, bei der alle 4 Gruppen der Massagekugeln den geringsten Abstand zueinander aufweisen und somit den minimalen Massagebereich definieren. In Längsrichtung beträgt der Achsabstand a' der außen liegenden Massagekugeln nunmehr 230 mm, der Achsabstand b' der außen liegenden Massagekugeln in Querrichtung 220 mm und der Achsabstand c' der innen liegenden Massagekugeln 130 mm. Beim Verschieben der Massagekugeln in die dargestellte Position, werden die Röhrenträger 14 und somit die UV-Röhren 10 über die Mitnehmer 13 in Pfeilrichtung y verschoben.

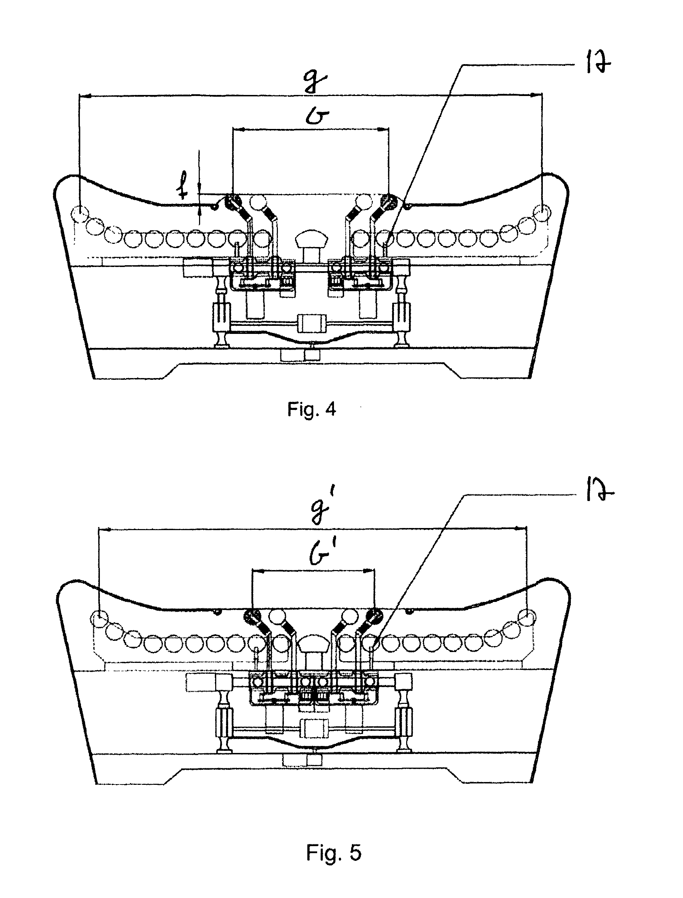

Fig. 4 und 5 zeigen jeweils einen Querschnitt durch die Liegefläche 3, wobei in Fig. 4 die Massagekugeln in auseinander gefahrener und angehobener Position und in Fig. 5 in zur Längsachse hin verschobener und abgesenkter Position dargestellt sind. Das Maß f in Fig. 4 bezeichnet das Maß der maximalen Anhebung der Massagekugeln in Bezug auf die Liegefläche 3. Das Maß f beträgt 20 mm. In Fig. 5 ist die Folie 7 bündig mit der Liegefläche 3. Aufgrund der Mitnehmer 13 variiert je nach Lage der Massagekugeln das Maß g, g', das den Achsabstand der jeweils außen liegenden UV-Röhren 10 angibt. In Fig. 4 beträgt das Maß g 830 mm, in Fig. 5 beträgt das Maß g' 770 mm. Die für das Mitführen der UV-Röhren 10 verantwortlichen Mitnehmer 13 stehen hierzu in Eingriff mit einem Führungsschlitz 17 des jeweiligen Röhrenträgers 14 und gewährleisten, dass bei einer horizontalen Verschiebung der Massageglieder 12 quer zur Längsachse der Liegefläche 3 die Röhrenträger 14 mit den hierauf angeordneten UV-Röhren 10 mitbewegt werden. Die Führungsschlitze 17 sind derart ausgebildet, dass eine Höhenverstellung der Massageglieder 12 auf die Lage der UV-Röhren 10 keinen Einfluss hat, d.h., dass in vertikaler Richtung die Führungsschlitze 17 ausreichend Bewegungsfreiraum für ein Heben und Absenken der Mitnehmer 14 besitzen. Den in den Fig. 4 und 5 dargestellten Umrissen ist weiterhin zu entnehmen, dass auch im Querschnitt die Liegefläche 3 ergonomisch geformt ist und eine Art Wanne bildet.

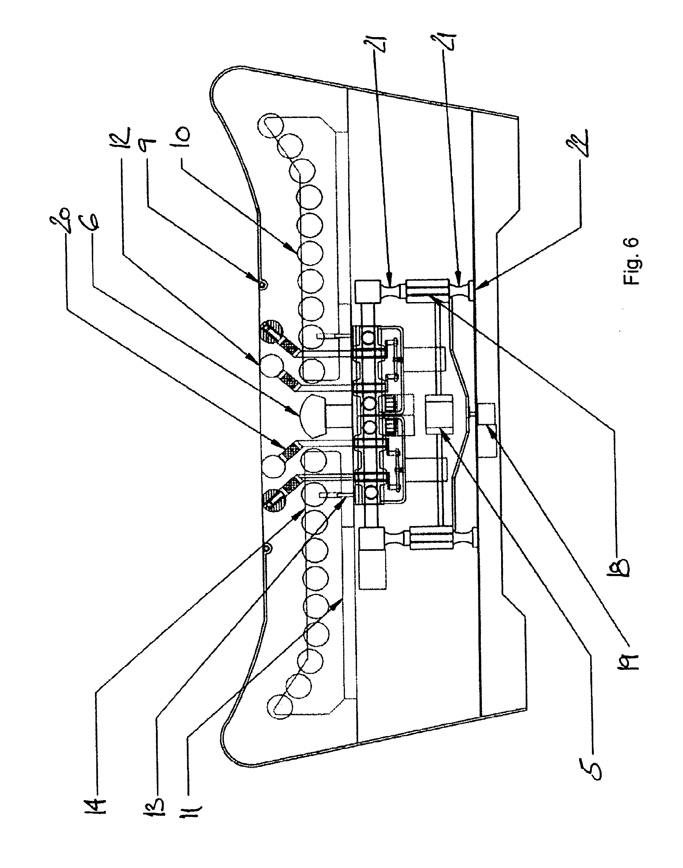

Fig. 6 stellt eine Vergrößerung des Querschnittes gemäß Fig. 5 dar. Die Bestrahlungseinheiten 1 umfassen jeweils 10 UV-Röhren 10, die auf quer zu den Röhrenlängsachsen ausgerichteten Röhrenträgern 14 angeordnet sind, wobei die jeweils außen liegenden drei Röhren, stufenweise angehoben sind und somit dem ergonomischen Verlauf der Liegefläche 3 folgen. Unterhalb der Röhrenträger 14 liegen Führungsschienen 11, die Gleitlager bilden, welche ein Verschieben der Röhrenträger 14 in einer horizontalen Ebene quer zur Längsachse der Liegefläche 3 ermöglichen. Zumindest ein Röhrenträger 14 einer jeden Bestrahlungseinheit 1 weist an seiner Unterseite einen Führungsschlitz 17 zur Aufnahme eines Mitnehmers 13 auf, der mit der Massageeinheit 2 verbunden ist.

Die Massageeinheit 2 umfasst ferner verschiedene Antriebseinheiten 5, u.a. mindestens einen Antrieb für eine Spindelhubeinheit 18 zur Höhenverstellung der Massageglieder 12 und eine Vibrationseinheit 19, mittels derer die auf Federelementen 20 gelagerten Massageglieder 12 in Schwingung versetzbar sind. Zur Übertragung der Schwingungen der Vibrationseinheit 19 weist die Massageeinheit 2 zudem Schwingelemente 21 auf.

Die Spindelhubeinheit 18 ermöglicht zudem eine Voreinstellung der Neigung der Massageeinheit 2, indem im Nacken- und im Lendenwirbelbereich getrennte Antriebe 5 vorgesehen sind und somit eine unterschiedliche Höhenlage der dort angeordneten Massagekugeln einstellbar ist.

Ferner weist die Massageeinheit 2 im Lagerbereich elektronische Druckaufnehmer 22 auf, mittels derer ein voreingestellter Massagedruck überwacht werden kann. Übersteigt der Massagedruck einen zuvor bestimmten Maximalwert bzw. sinkt unter einen zuvor bestimmten Minimalwert, kann über die Spindelhubeinheit 18 die Höhenlage der Massageglieder 12 derart verändert werden, dass ein optimaler Massagedruck anliegt. Eine programmierbare Steuerung (nicht dargestellt) regelt diesen Vorgang.

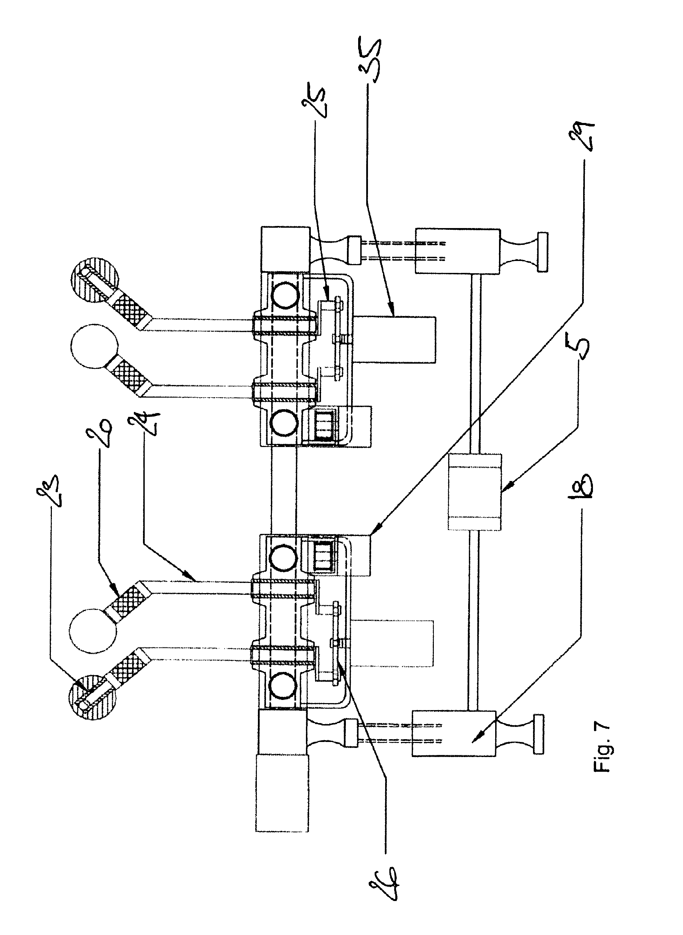

Fig. 7 zeigt einen Querschnitt durch die Massageeinheit 2 entsprechend Fig. 6 in einem größeren Maßstab. Eine Drehbewegung der Massagekugeln ermöglichen stiftförmige Rotationslager 23, auf die die Massagekugeln aufgesteckt sind. Zum Wechseln der Massagekugeln, beispielsweise um eine Kugel mit einer anderen Oberfläche aufzustecken, können diese durch einfaches Abziehen abgenommen werden. Der Durchmesser der Kugeln beträgt vorliegend 30 mm. Die Rotationslager 23 sind in einem Anstellwinkel von etwa 45° auf einem Wellenschaft 24 angeordnet, der wiederum über einen Kurvenhebel 25 mit einem Schieber 26 verbunden ist. Jeder Massagekugelgruppe ist ein Schieber 26 zugeordnet, so dass die zur jeweiligen Gruppe gehörenden Massagekugeln die durch den Schieber 26 ausgelösten Bewegungen synchron ausführen.

Zwischen dem Rotationslager 23 und dem Wellenschaft 24 eines Massagegliedes 12 ist das Federelement 20 angeordnet. Das Federelement 20 ermöglicht ein gleichmäßiges Abrollen der Massagekugel entlang der zu massierenden Körperzone, indem es einen gleichmäßigen Anpressdruck der Massageglieder 12 an die - im Normalfall unregelmäßig geformte - Körperzone gewährleistet. Ferner wird ein gleichmäßiges Abrollen durch den vorgesehenen Anstellwinkel von etwa 45° unterstützt.

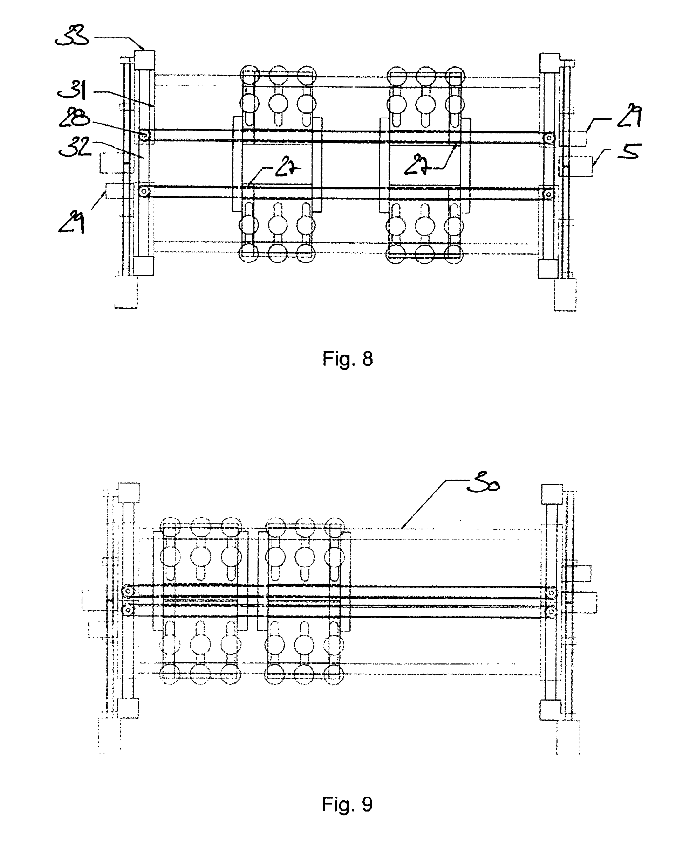

Jeweils zwei Massagekugelgruppen bilden eine obere und eine untere Einheit (siehe Fig. 8 und 9), die getrennt voneinander in einer horizontalen Ebene parallel zur Längsachse der Liegefläche 3 verschiebbar sind. Der Antrieb erfolgt über zwei getrennte, über Umlenkrollen 28 geführte Zahnriemen 27, denen jeweils ein eigener Antrieb 29 zugeordnet ist. Jede Einheit ist in einem Zahnriemenfixpunkt mit dem zugehörigen Zahnriemen 27 verbunden.

Fig. 8 und 9 ist ebenfalls zu entnehmen, dass die Massageeinheit 2 parallel zur Längsachse der Liegefläche 3 verlaufende Führungsstangen 30 aufweist, welche paarweise über Gleitbuchsen 31 mit quer zur Längsachse der Liegefläche verlaufende, seitliche Führungsstangen 32 verbunden sind. Jeweils ein längsaxial verlaufendes Führungsstangenpaar bildet mit den entsprechenden Gleitbuchsen einen Schlitten, über den die Massagekugelgruppen paarweise in einer horizontalen Ebene quer zur Längsachse verschoben werden können. Die seitlichen, quer zur Längsachse verlaufenden Führungsstangen 32 sind über Knotenstücke 33 mit der Spindelhubeinheit 18 verbunden, so dass die obere seitliche Führungsstange 32 über einen oberen Antrieb der Spindelhubeinheit 18 getrennt von der unteren Führungsstange, welche entsprechend mit einem unteren Antrieb der Spindelhubeinheit 18 verbunden ist, in vertikaler Richtung anhebbar und absenkbar ist.

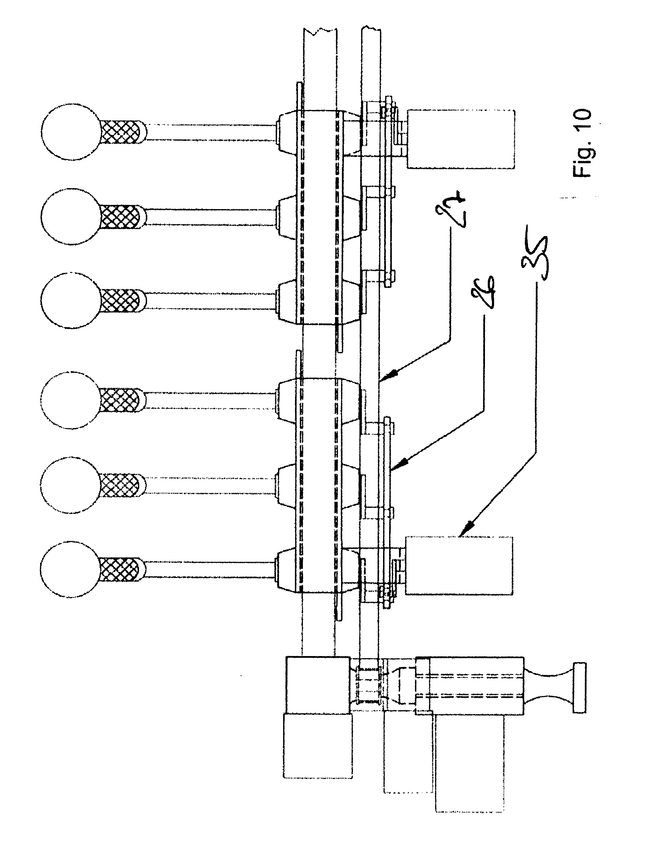

Fig. 8 zeigt die obere und die untere Einheit in einer zueinander beabstandeten Position, wobei die Schlitten auseinander gefahren sind, so dass das Gruppenpaar einer Einheit zueinander beabstandet ist. Fig. 9 zeigt eine Anordnung, bei der die Schlitten und die obere und untere Einheit eng beieinander liegen. Fig. 10 gibt einen Längsschnitt durch die Massageeinheit 2 wieder, wobei die Massageglieder 12 die Anordnung entsprechend Fig. 9 aufweisen.

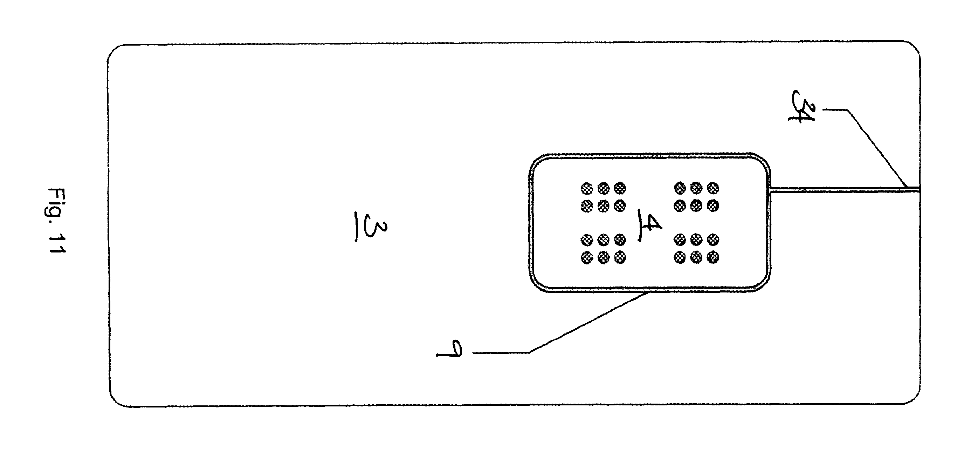

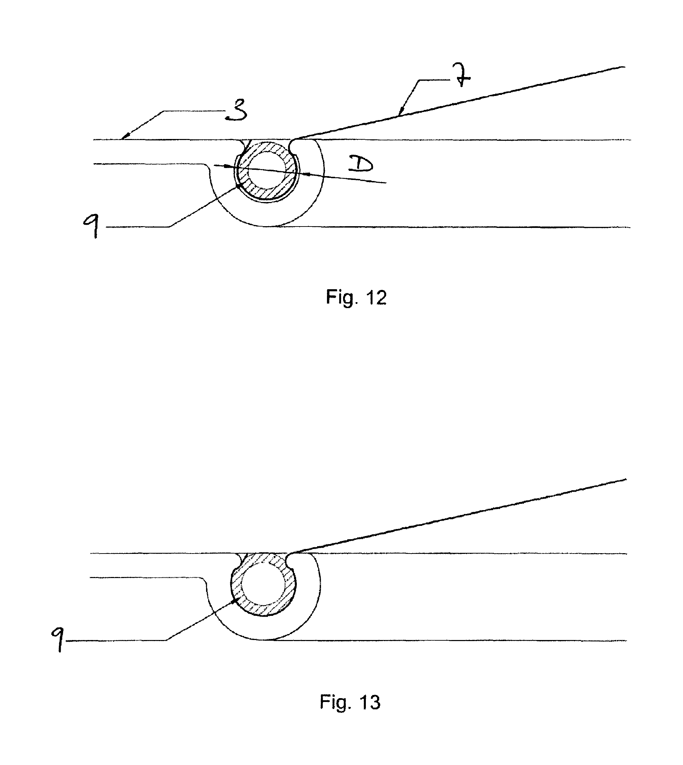

Fig. 11 stellt eine weitere Draufsicht auf die Liegefläche 3 dar, wobei nur der Umriss der Liegefläche und der elastische strahlungsdurchlässige Bereich 4 einschließlich Führung der Schlauchringleitung 9 wiedergegeben sind. Der elastische strahlungsdurchlässige Bereich 4 und die darunter liegende Massageeinheit 2 sind derart auf der Liegefläche 3 angeordnet, dass eine in Rückenlage aufliegende Person von den Lendenwirbeln an aufwärts bis zum Nackenbereich massiert werden kann. Der elastische strahlungsdurchlässige Bereich 4 besteht aus einer reißfesten, UV-durchlässigen Folie 7, die eine dem Bereich 4 entsprechende Aussparung in der Liegefläche 3 überspannt. Die Folienränder sind hierzu in einer umlaufenden Ausnehmung 8 mit einem annähernd kreisförmigen Querschnitt mit einem Durchmesser D von 8 mm eingelegt und werden über eine mit Druckluft beaufschlagte Schlauchringleitung 9 abdichtend gegen die Innenwände der umlaufenden Ausnehmung 8 gedrückt (siehe Fig. 12 und 13). Eine Stichleitung 34 führt von der Schlauchringleitung 9 zu einem Druckluftanschluss, der in Fig. 11 nicht dargestellt ist.

Zum Wechseln der Folie 7, was bei Bedarf nach jeder Behandlung erfolgen kann, wird die Schlauchringleitung 9 in entspanntem Zustand, d.h. ohne Druckluftbeaufschlagung (siehe Fig. 12), entnommen und die Folie 7 gegen eine neue Folie ausgetauscht. Danach wird die Schlauchringleitung 9 wieder eingelegt und mit Druckluft beaufschlagt (siehe Fig. 13). Der Anpressdruck der Schlauchringleitung 9 gewährleistet eine feuchtigkeitsundurchlässige Verbindung der Folie 7 mit der Liegefläche 3. Zugleich sichert der Anpressdruck ein Verrutschen der Folie. Die druckbeaufschlagte Schlauchringleitung 9 schließt bündig mit der Liegefläche 3 ab, so dass der Liegekomfort nicht beeinträchtigt wird.

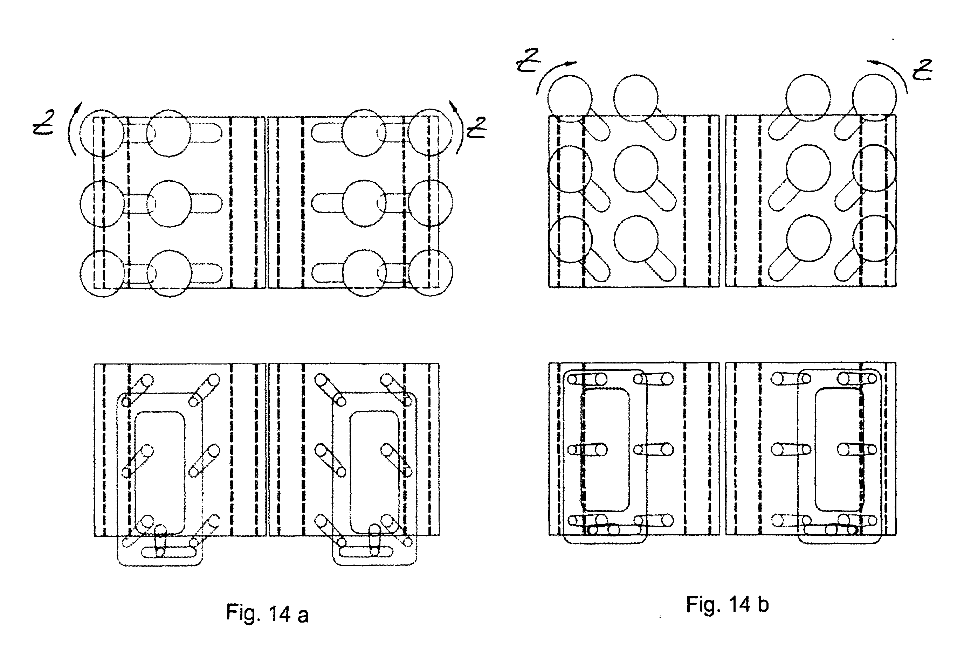

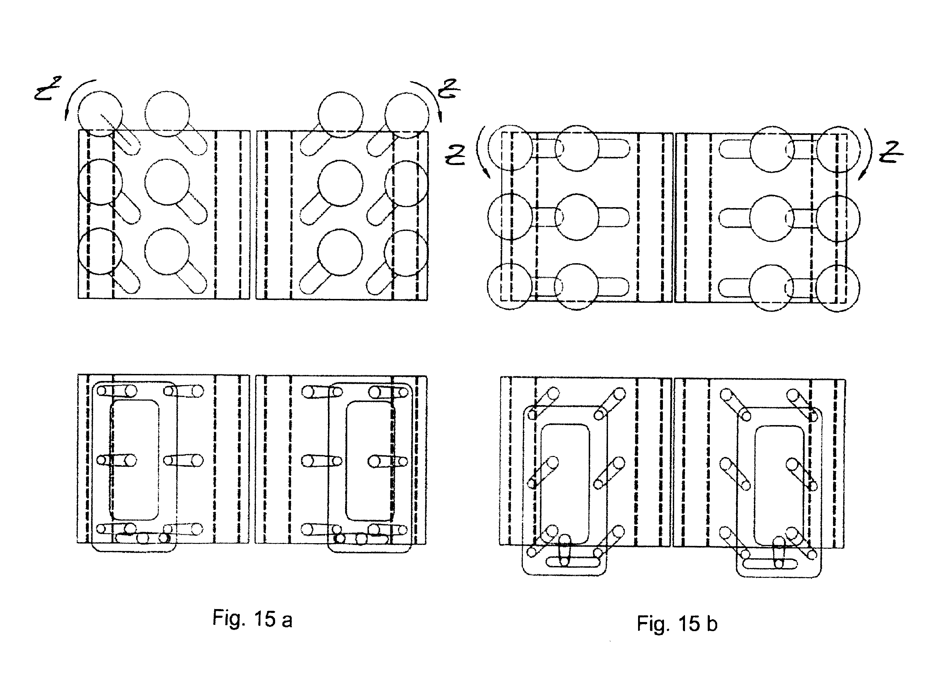

Die in den Fig. 14 und 15 enthaltenen schematischen Darstellungen zeigen mögliche Bewegungsabläufe der Massagekugeln. Fig. 14a zeigt die Position der Massagekugeln (obere Darstellung) bzw. der Kurvenhebel 25 und Schieber 26 (untere Darstellung) in der Grundstellung. Noch in der Grundstellung wird zunächst durch Verschieben der Schlitten quer zur Längsachse der Abstand der Massagekugeln auf die zu massierende Rückenbreite eingestellt. Durch Verschieben der oberen und unteren Einheit parallel zur Längsachse werden dann die Massagekugeln in Bewegung versetzt. Währendessen wird der optimale Massagedruck über eine entsprechende Höhenverstellung der Massagekugeln eingestellt. In einer vorbestimmten Position verharren dann obere und untere Einheit und die Massagekugeln werden gruppenweise um die Längsachse ihrer Wellenschäfte 24 verschwenkt (Pfeil z). Dabei können die Schwenkbewegungen gruppenweise je nach Massageprogramm gleich- oder gegenläufig ausgeführt werden.

Die Massagekugeln sind ausgehend von der Grundstellung in beide Richtungen um etwa 45° horizontal verschwenkbar (siehe Fig. 14b). Fig. 15a und 15b zeigen die Schwenkbewegung zurück in die Grundstellung und die entsprechende Stellung der Kurvenhebel 25 und Schieber 26. Das Verschwenken der Massagekugeln wird durch einen mit dem jeweiligen Schieber 26 einer Massagekugelgruppe verbundenen Exzenterantrieb 35 ausgelöst. Bevorzugt führen die Massagekugeln die Schwenkbewegung bei Stillstand der oberen und unteren Einheit aus, damit ein einwandfreies Abrollen der Massagekugeln gewährleistet werden kann.

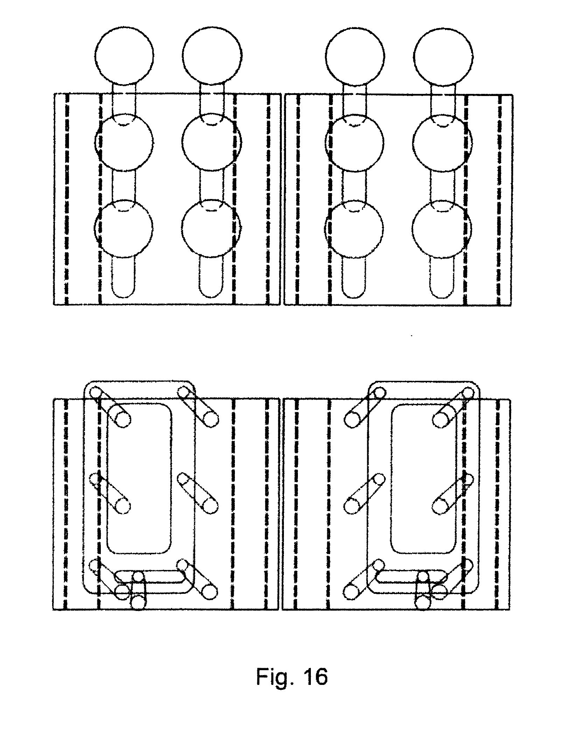

Fig. 16a zeigt die Stellung der Massagekugeln nach Beendigung einer Behandlung. Die Massagekugeln werden in eine Parkposition verfahren, in der sie in der Lage sind, den Körper zu stützen, um das Absteigen von der Liegefläche bzw. das Aufsteigen zu erleichtern. Fig. 16b zeigt die entsprechenden Kurvenhebel- und Schieberpositionen.

### Bezugszeichenliste

- 1: Bestrahlungseinheit
- 2: Massageeinheit
- 3: Liege- oder Sitzfläche
- 4: Elastischer strahlungsdurchlässiger Bereich
- 5: Antriebseinheit
- 6: Strahlerleiste
- 7: Folie
- 8: Ausnehmung
- 9: Schlauchringleitung
- 10: Strahlungsemittierende Röhre
- 11: Führungsschienen
- 12: Massageglieder
- 13: Mitnehmer
- 14: Röhrenträger
- 15: Fußteil
- 16: Kopfteil
- 17: Führungsschlitz
- 18: Spindelhubeinheit
- 19: Vibrationseinheit
- 20: Federelemente
- 21: Schwingelemente
- 22: Druckaufnehmer
- 23: Rotationslager
- 24: Wellenschaft
- 25: Kurvenhebel
- 26: Schieber
- 27: Zahnriemen
- 28: Umlenkrolle
- 29: Zahnriemenantrieb
- 30: Führungsstange
- 31: Gleitbuchsen
- 32: Seitliche Führungsstange
- 33: Knotenstücke
- 34: Stichleitung
- 35: Exzenterantrieb

## Patentansprüche

1. Vorrichtung zur therapeutischen und/oder kosmetischen Behandlung einer Körperzone mit einer Bestrahlungseinheit (1) und einer Massageeinheit (2),
**dadurch gekennzeichnet, dass**
die Bestrahlungseinheit (1) und die Massageeinheit (2) zeitgleich auf die Körperzone einwirken.

2. Vorrichtung nach Anspruch 1, **dadurch gekennzeichnet, dass** die Vorrichtung eine Liege- oder Sitzfläche (3) umfasst, in der die Bestrahlungseinheit (1) und die Massageeinheit (2) integriert sind.

3. Vorrichtung nach Anspruch 1 oder 2, **dadurch gekennzeichnet, dass** die Liege-oder Sitzfläche (3) mindestens einen elastischen und/oder strahlungsdurchlässigen Bereich (4) aufweist, hinter dem die Bestrahlungseinheit (1) und die Massageeinheit (2) ganz oder teilweise angeordnet sind.

4. Vorrichtung nach einem der Ansprüche 1 bis 3, **dadurch gekennzeichnet, dass** die Bestrahlungseinheit (1) mindestens eine strahlungsemittierende Röhre (10) und/oder mindestens einen Strahler umfasst, die bzw. der beweglich gelagert und/oder auf die zu behandelnde Körperzone ausrichtbar ist.

5. Vorrichtung nach einem der Ansprüche 1 bis 4, **dadurch gekennzeichnet, dass** die Massageeinheit (2) mindestens ein beweglich gelagertes und/oder auf die zu behandelnde Körperzone ausrichtbares Massageglied (12) umfasst, mittels dessen Druck auf die zu behandelnde Körperzone ausübbar ist.

6. Vorrichtung nach Anspruch 5, **dadurch gekennzeichnet, dass** das Massageglied (12) mit mindestens einer elektrischen Antriebseinheit (5) verbunden ist, mittels derer das Massageglied (12) in lineare, insbesondere rollende oder klopfende und/oder kreisende und/oder oszillierende Bewegungen versetzbar ist.

7. Vorrichtung nach einem der Ansprüche 2 bis 6, **dadurch gekennzeichnet, dass** hinter zumindest einem elastischen und/oder strahlungsdurchlässigen Bereich (4) der Liege- oder Sitzfläche (3) zusätzlich mindestens eine breit abstrahlende Strahlerleiste (6) angeordnet ist, deren Strahlung Bereiche geringerer Strahlungsintensität erreicht.

8. Vorrichtung nach einem der Ansprüche 2 bis 7, **dadurch gekennzeichnet, dass** mindestens ein elastischer und/oder strahlungsdurchlässiger Bereich der Liege-oder Sitzfläche (3) aus einer abnehmbaren Folie (7) besteht, die feuchtigkeitsundurchlässig mit der Liege- oder Sitzfläche verbunden ist.

9. Vorrichtung nach Anspruch 8, **dadurch gekennzeichnet, dass** um den elastischen und/oder strahlungsdurchlässigen Bereich in der Liege- oder Sitzfläche (3) eine Ausnehmung (8) zur Aufnahme des Folienrandes angeordnet ist, wobei zur Fixierung des Folienrandes zusätzlich eine mit Druckluft beaufschlagbare Schlauchringleitung (9) in die Ausnehmung (8) einlegbar ist.
